Europäisches Patentamt

European Patent Office

Office européen des brevets

(11) Publication number: **0 402 467**
**A1**

## EUROPEAN PATENT APPLICATION
## published in accordance with Art. 158(3) EPC

(21) Application number: **89902294.1**

(22) Date of filing: **10.02.89**

(86) International application number:
**PCT/JP89/00134**

(87) International publication number:
**WO 89/07413 (24.08.89 89/20)**

(51) Int. Cl.5: **A61B 1/00**

(30) Priority: **15.02.88 JP 32137/88**

(43) Date of publication of application:
**19.12.90 Bulletin 90/51**

(84) Designated Contracting States:
**BE DE FR GB IT NL SE**

(71) Applicant: **Terumo Kabushiki Kaisha**
**No. 44-1, Hatagaya 2-chome Shibuya-ku**
**Tokyo 151(JP)**

(72) Inventor: **HONDA, Hiroaki**
**1-31, Sobe 1-chome Naha-shi**
**Okinawa 900(JP)**

(74) Representative: **Casalonga, Axel et al**
**BUREAU D.A. CASALONGA - JOSSE**
**Morassistrasse 8**
**D-8000 München 5(DE)**

(54) **CATHETER TUBE AND ENDOSCOPE.**

(57) When an observation unit is attached to a catheter tube having a first inflatable balloon on a tube body and a second balloon which is set uninflated in the tube body and can be extended from the tube body to be inflated, the catheter tube can be used as an endoscope for observing the interior of a body cavity and giving a medical treatment thereto from a position outside the body. This tube is capable of defining an observation space of an arbitrary length between the first and second balloons, and preventing the entry of a body fluid, such as blood, thereinto. This enables a transparent visual field to be obtained.

FIG. 6

SPECIFICATION

## TITLE OF THE INVENTION

CATHETER TUBE AND ENDOSCOPE

## Technical Field

This invention relates to a catheter tube that is used in the state of being inserted into the body cavity, especially the blood vessel, for observing the inside of the body cavity and/or performing a therapeutic treatment of the inner wall surface of the body cavity from the position out of the patient's body, and an endoscope (a fiberscope) employing the catheter tube.

## Background Art

With an endoscope, the inside of the body cavity into which it is introduced may be observed from the position outside the patient's body, while therapeutic treatment, such as administration of the liquid drug to the inner wall of the body cavity or irradiation of the laser light, may be performed, so that it is attracting attention in recent years and its evolution is progressing.

The inside of the blood vessel may be observed with the endoscope after the blood obstructing the field of view is displaced at the site of observation.

-2-

In displacing the blood in this manner, it has been customary to inflate a balloon formed around the outer periphery of the tube to interrupt the blood flow, to eject the transparent liquid, such as physiological saline, into the region to be observed to displace the blood and to cause the region to be filled with the transparent liquid.

However, with this method, blood flow reversal may be caused in the course of the observation in the artery, while the blood tends to flow into the transparent field of view by way of branching sites of the blood vessel ahead and at back of the balloon, so that the transparent field of view cannot be obtained stably.

Disclosure of the Invention

It is an object of the present invention to solve the above mentioned deficiency of the prior art and to provide a catheter tube adapted to prevent blood inflow to the target site in the blood vessel and an endoscope making use of the catheter tube.

This object may be achieved by the present invention.

Thus the present invention provides a catheter tube characterized in that the catheter tube comprises a tube main body,

an inflatable first balloon placed around an outer peripheral wall of the tube in the vicinity of a distal end of the tube main body,

a first lumen formed in the tube main body and communicating with an interior of said first balloon,

a tubular member provided in the vicinity of a distal end thereof with an inflatable second balloon and having a passage communicating with the interior of said second balloon, and

a second lumen formed in said tube main body and adapted for accommodating said tubular member therein,

wherein said tubular member moves axially within said second lumen with said second balloon remaining in the deflated state so that said second balloon can be moved to a position spaced a predetermined distance from the distal end of said tube main body.

The constituent material of the tubular member is preferably an ultraelastic alloy.

The tubular member is preferably used as a guide wire for the catheter tube.

The present invention also provides a catheter tube characterized in that the catheter tube comprises a tube main body,

an inflatable first balloon placed around the outer peripheral wall of the tube in the vicinity of the distal end of the tube main body,

-4-

a lumen formed in said tube main body, said lumen communicating with the inside of said first balloon and opening into the foremost part of the tube main body, and

a tubular member accommodated within said lumen, said tubular member having an inflatable second balloon in the vicinity of the distal end thereof and a passage communicating with the interior of said second balloon,

wherein said tubular member moves axially within said lumen with said second balloon remaining in the deflated state so that said second balloon can be moved to a position spaced a predetermined distance from the distal end of said tube main body.

The above described catheter tube of the present invention is preferably a catheter tube for blood vessel.

The present invention also provides an endoscope in which the tube main body of the catheter tube is provided with a lumen adapted for accommodating an instrument for observation and the instrument of observation is accommodated within the lumen.

Brief Description of the Drawings

Fig. 1 is a partial longitudinal cross-sectional view showing a constructional example of the catheter tube and the endoscope according to the present invention.

Fig. 2 is a cross-sectional view taken along line II - II of Fig. 1.

Fig. 3 is a longitudinal cross-sectional view showing a constructional example of the tubular member in the present invention.

Figs. 4, 5 and 6 are partial cross-sectional side views showing examples of using the catheter tube (endoscope) of the present invention.

Best Embodiment for Practicing the Invention

The catheter tube and the endoscope according to the present invention will be explained by referring to preferred embodiments thereof shown in the accompanying drawings.

Although the application of the catheter tube of present invention to an endoscope is representatively explained herein below, the catheter tube of the present invention is not limited thereto, but may also be used in a urethral catheter, a catheter for laser therapy or a biliary catheter.

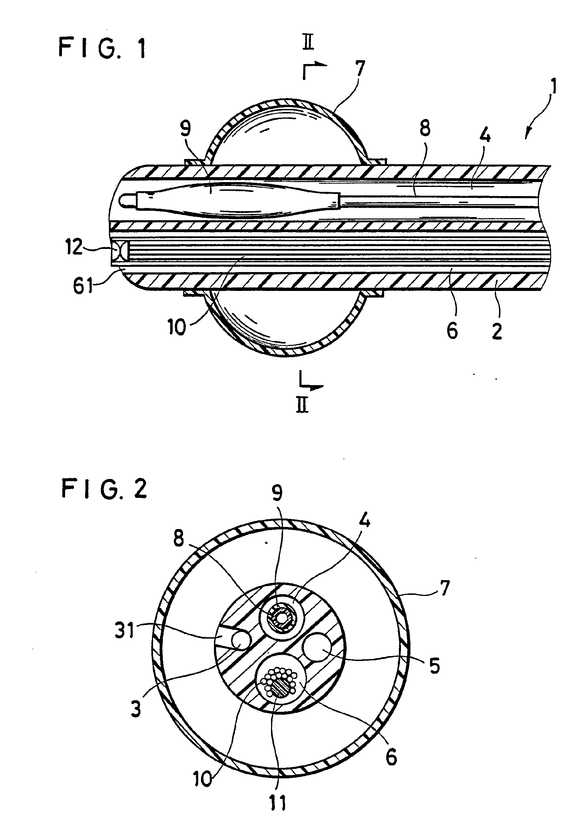

Fig. 1 is a partial longitudinal cross-sectional view showing constructional examples of the catheter tube and the endoscope according to the present invention and Fig. 2 is a cross-sectional view taken along line II - II of Fig. 1.

As shown in these figures, a catheter tube 1 has a main tube body 2, and first balloon 7 is provided around the outer peripheral wall of the tube main body in the vicinity of the distal end thereof shown towards left in Fig. 1.

This first balloon 7 is formed of rubber, such as silicone rubber or latex rubber, urethane, PVC, EVA, or polyethylene terephthalate, for example, and may be inflated or deflated freely.

The tube main body 2 is formed of a flexible material, such as PVC, polyurethane, silicone rubber, PE, nylon or EVA.

The tube main body 2 is formed with various lumens of different usages and functions, as will be explained subsequently.

A first lumen 3 communicates with the first balloon 7 via a side hole 31 for supplying an actuating fluid, which may be a gas or a liquid, into the first balloon 7 to inflate the first balloon 7, or discharging the actuating fluid out of the first balloon 7 to deflate the first balloon 7.

The first balloon 7 is adapted to be contacted tightly with the inner wall surface of the body cavity into which the catheter is introduced, when the balloon is inflated, and plays the role of securing the catheter tube 1 with respect to the body cavity and the role of displacing the blood obstructing the field of view ahead of the first balloon 7 or towards the distal end of the tube main body and of interrupting blood inflow into the field of view during blood replacement by a transparent liquid.

Preferably, the first balloon 7 is adapted to be inflated radially from the center of the tube main body 2.

Although the first balloon 7 may be of a circular, elliptical or similar cross-section, it is preferably of a cross-section similar to the cross-section of a body cavity into which it is to be introduced and retained for intimate contact with the inside of the body cavity.

Meanwhile, the first balloon 7 needs to be attached to the tube main body 2 air-tightly or liquid-tightly. As attaching means for the first balloon 7, a separate member, such as a cylindrical or pouch-shaped rubber member, may be bonded or affixed to the main tube body by an adhesive or a yarn, respectively or the balloon may be formed integrally with the tube main body by monolithic molding or two color molding, provided that air tightness of the balloon may be guaranteed by the attachment means employed.

It is noted that the first balloon 3 may be opened towards the foremost part of the tube main body 2 so as to be used simultaneously as a lumen for ejecting the transparent liquid, which with be explained subsequently, or as fiber accommodating lumen.

A second lumen 4 is opened at the foremost part of the tube main body 2 and, within this lumen, a tubular member 8 provided with a second balloon 9 is accommodated so as to be movable along the axis of the tube main body.

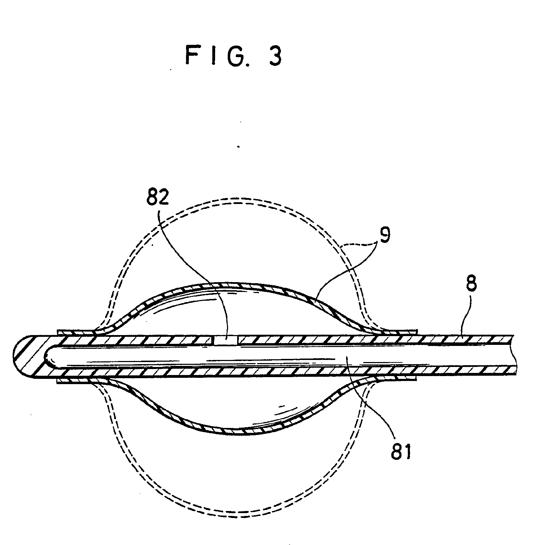

Referring to Fig. 3, the tubular member 8 is a hollow tube occluded at the foremost part thereof which is the lefthand side in the drawing. A passage 81 is formed within the tubular member, while a side hole 82 is formed in the side wall of the tubular member 8 for establishing communication between the passage 81 and the inside of the second balloon 9. The second balloon 9, which may be inflated and deflated freely, is mounted around the outer peripheral wall of the foremost part of the tubular member 8 for covering the side hole 82 and, when the actuating fluid is supplied from the proximal end of the tubular member 8 into the passage 82, the actuating fluid flows into the second balloon 9 via passage 81 and side hole 82 to inflate the second balloon 9 as indicated by broken lines in Fig. 3.

Similarly to the above-mentioned first balloon 7, the second balloon 9 is also adapted to be contacted, when inflated, with the inner wall surface of the body cavity into which the catheter tube 1 is introduced, and plays the role of interrupting blood influx into the field of sight when the transparent liquid is substituted for blood. The constituent material and the shape as well as mounting means for the second balloon 9 are the same as those explained with reference to the above-mentioned first balloon 7.

The constituent material for the tubular member 8 may be arbitrarily selected provided that these are soft and pliable while being excellent in torque transmitting properties. Examples of these materials include metals such as nickel-titanium alloys, stainless steel or platinum and various plastics such as polypropylene, vinyl chloride or nylon. Of these, ultraelastic alloys such as nickel-titanium alloys are most preferred because the tubular member 8 then may be used as a guide wire.

The tubular member 8 is moved within the second lumen 4, with the second balloon 9 remaining in the deflated state, and is stopped at a position which is ahead of the site of observation and which spaced a predetermined distance from the distal end of the main tube body 2. The second balloon 9 is inflated at this position. The tubular member 8 is moved a distance so that the spacing between the first and the second balloons is about 10 to 30 mm, as an example. The tubular member 8 may be moved by any desired means and, for example, a plunger may be connected to the proximal end of the tubular member 8 which may be moved a desired distance by the plunger operation.

The surface of the tubular member and/or the inner surface of the second lumen 4 may be subjected to suitable lubricating treatment to permit smooth movement of the tubular member 8 within the second lumen 4.

By way of lubricating treatment, coating of a lubricant such as silicone oil, Teflon, olive oil, glycerin or polyethylene glycol or hydrophilizing treatment may be employed.

A third lumen 5 is opened at the foremost part of the tubular main body 2 at an opening 51 of which the fluid may

-10-

be introduced into or sucked out of the body cavity. More specifically, the third lumen 5 may be used for administering a liquid drug or the like into the body cavity in which the catheter tube 1 has been inserted and retained, or may be used as a flushing channel by means of which a transparent liquid, such as a physiological saline, for displacing the blood otherwise obstructing the field of view at the time of observation of the inside of the blood vessel by the endoscope.

A fourth lumen 6 is opened at the foremost part of the tube main body 2, and a bundle of optical fibers, as an instrument for observing within the fourth lumen for constituting the endoscope of the present invention.

This optical fiber bundle is constituted by light transmitting fibers or a light guide 10 and light receiving fibers or imaging fibers 11, with the foremost part of the optical fibers lying in the vicinity of an end opening 61 of the fourth lumen 6. A lens 12 is attached to the foremost part of the optical fibers 10, 11.

The light radiated from a light source, not shown, at the proximal end of the catheter tube 1 towards the righthand side of Fig. 1, is transmitted within the inside of the light transmitting optical fibers 10 so as to be irradiated on the site of observation via the forward end of the optical fibers. The reflected light is introduced at the foremost part of the light receiving fibers 11 and the corresponding image is transmitted through the inside of the fibers 11 to an image receiving section, not shown, at the proximal end of the catheter tube.

In general, these light transmitting and light receiving fibers are constituted by optical fibers of, for example, quartz, plastics or multi-component glasses.

With the catheter tube of the present invention, the third lumen 5 and/or the fourth lumen 6 need not be provided, depending on the usage of the catheter tube. Alternatively, one or more lumens may be provided which will be used simultaneously as one or more of the first balloon inflating lumen, tubular member accommodating lumen, flushing lumen or the fiber accommodating lumen. Lumens having usages and functions other than those described above may also be annexed, if so desired.

Example 1

A catheter tube having the construction as shown in Figs. 1 and 2 was prepared. The following are various conditions for the catheter tube.

Tube Main Body

Constituent Material : vinyl chloride with X-ray
contrast medium

Outside Diameter : about 2.5 mm

Overall Length : about 1.5 m

Lumens : four lumens

First Balloon inflating Lumen (inside diameter, 0.3 mm) Tubular Member Accommodating Lumen (inside diameter, 1.0 mm)
(lubricated by silicone coating)

Transparent Liquid Ejecting Lumen (inside diameter, 0.3 mm) Fiber Accommodating Lumen (inside diameter, 0.8 mm) First Balloon

Constituent Material : Latex Rubber

Thickness : 0.1 mm

Shape : Cylindrical

Effective Length : 7 mm

Diameter of Inflatable Section : 1.9 mm (on deflation) and 6 mm (on inflation)

Second Balloon

Constituent Material : Latex Rubber

Thickness : 0.1 mm

Shape : Cylindrical

Effective Length : 7 mm

Diameter of Inflatable Section : 0.6 mm (on deflation) and 6 mm (on inflation)

Tubular Member

Constituent Material : ultraelastic alloy (composition :

nickel·titanium)

Outside Diameter : 0.46 mm

Inside Diameter of Passage : 0.2 mm

Imaging fibers (about 2000 quartz fibers, each about 2 to 3 μm, bundled together) and the light guide (about 25 quartz fibers, each about 50 μm, bundled together) were unified to a fiber bundle having an outside diameter of about 0.5 mm Ø, and were introduced into the fiber accommodating lumen of the catheter tube to produce the endoscope.

A convex lens was attached to the end faces of the imaging fibers for receiving the light irradiated from the light guide and forming the image of the object on the end faces of the imaging fibers.

A video camera was attached to the proximal ends of the imaging fibers to permit the image to be observed by a monitor TV. A light connector was attached to the proximal end of the light guide and connected to a white light source.

The tubular member was adapted to be inserted into and extracted from the tubular member accommodating lumen.

A valve was mounted at a socket for the tubular member of the tubular member accommodating lumen.

A stopper having a Luer tapered socket was attached to the distal end of the transparent liquid ejecting lumen and a syringe A was connected thereto to permit a physiological saline to be supplied to the lumen.

A valve having a Luer tapered socket was attached to each of the lumen communicating with the first balloon and to the proximal end of the passage communicating with the second

-14-

balloon and syringes B and C are connected thereto to enable the fluid for inflation, such as $CO_2$ gas, to be injected into each of the balloons.

The above described endoscope was inserted as far as the target site in the blood vessel with the inside diameter of approximately 5 mm. The tubular member remained in the state in which it was introduced into the lumen up to the position is which the second balloon was contacted with the end face of the tubular main body.

The syringe B was first actuated for injecting the $CO_2$ gas for inflating the first balloon to secure the endoscope with respect to the blood vessel as well as to interrupt the blood stream.

The tubular member was then gripped and moved towards the distal end of the tube until the distance of approximately 30 mm was reached between the first and the second balloons. The syringe A was then actuated for injecting 5 ml of physiological saline into the blood vessel ahead of the first balloon (at the forward side end) while displacing the blood and charging the physiological saline.

The syringe C was then actuated for injecting the $CO_2$ gas for inflating the second balloon into tight contact with the inner wall of the blood vessel.

The space between the first and the second balloons was filled with physiological saline to form a transparent field of view.

In this state, the light was irradiated from the light guide and the image of the inner wall surface of the blood vessel, displayed via the imaging fibers, was observed on the monitor television. There was no blood inflow to the site of observation and a clear image could be observed for several minutes.

## Operation of the Invention

The operation of the catheter tube and the endoscope according to the present invention will be explained herein below.

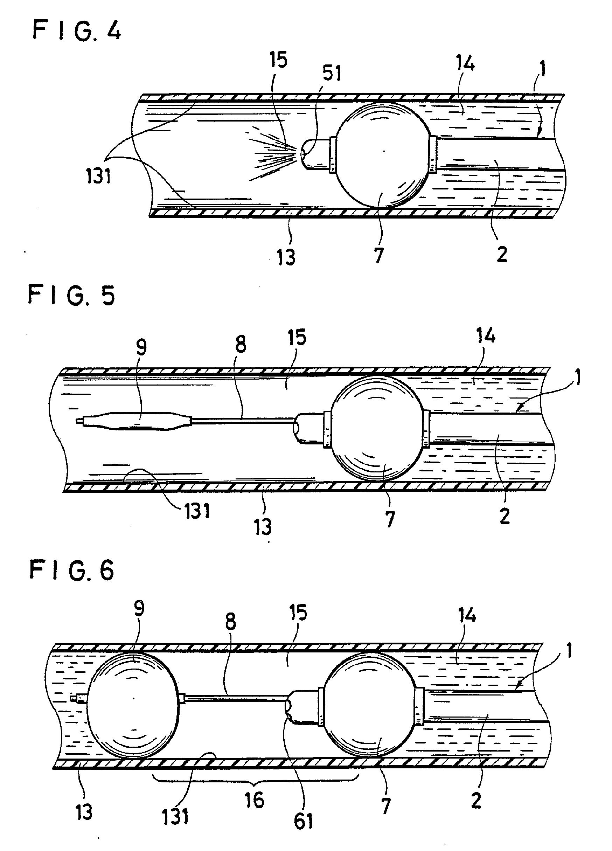

Figs. 4, 5 and 6 are partial cross-sectional side elevational views showing the state of use of the endoscope constituted by the catheter tube of the present invention.

Referring to Fig. 4, the catheter tube 1 is inserted into the body cavity, that is into blood vessel 13, and an actuating fluid is introduced from the first lumen 3 into the first balloon 7 to inflate the first balloon 7. The first balloon 7 is brought into tight contact with the inner wall surface 131 of the blood vessel 13 to secure the catheter tube 1 with respect to the blood vessel 13 as well as to interrupt the flow of blood 14 towards both left and right in drawing.

-16-

In this state, a transparent liquid 15, such as physiological saline, is ejected via opening 51 at the foremost part of the tube and via third lumen 5 for thrusting and displacing the blood ahead of the first balloon (towards the foremost part) for substitution thereof by the transparent liquid 15.

Then, as shown in Fig. 5, the tubular member 8 accommodated within the second lumen 4 is moved towards the forward side of the tube so that the second balloon 9 attached to the foremost part of the tubular member 8 in the deflated state is placed at a position which is more forward than the observation site and which is spaced a predetermined distance from the foremost part of the tube main body 2. It is noted that ejection of the transparent liquid 15 and movement of the tubular member 8 may be effected simultaneously or in the sequence which is reversed from the above described sequence.

Then, as shown in Fig. 6, the actuating fluid is supplied via passage 81 of the tubular member 8 into the second balloon 9 for inflating the second balloon 9 into tight contact with the inner wall surface 131 of the blood vessel 13. In this state, the transparent field of view 16 filled with the transparent liquid 15 is defined in a region between the first balloon 7 and the second balloon 9 within the blood vessel 13 to permit the inside of the blood vessel to be observed via opening 61 formed at the foremost part of the tube main body 2 and by way of the light transmitting fibers 10 and the light receiving fibers 11.

The site of observation in the blood vessel 13 is delimited by the first and the second balloons 7, 9 kept in tight contact with the inner wall surface 131 of the blood vessel, so that a clear view may be had for a prolonged time without the blood 14 flowing into the transparent field of view 16 between the two balloons.

After termination of the observation, the sequence of operations reversed from that described above is performed, that is, the second balloon 9 is deflated, the tubular member 8 is moved towards the proximal end and accommodated within the second lumen 4. The first balloon 7 is then deflated and released.

It is to be noted that the usage of the catheter tube 1 of the present invention is not limited to the fiberscope for observing the inside of the blood vessel, but it may be applied to a wide field of applications, such as administration of liquid drugs, irradiation of a laser beam via optical fibers or to end guiding for insertion to a target site.

Possibility of Industrial Application

With the catheter tube and the endoscope according to the present invention, observation of the inside of the body cavity and therapeutic treatment may be performed within a space in the body cavity delimited by the first balloon and the second balloon, so that, when the inside of the blood vessel is observed by a fiberscope, by way of an example, a clear image can be viewed for a prolonged time, without the blood obstructing the field of view flowing into the transparent field of view (observation site) defined between the first balloon and the second balloon.

On the other hand, since the tubular member provided with the second balloon may be moved along the axis of the catheter, the region from which to take the clear view may be selected in a desired manner.

In addition, the tubular member provided with the second balloon may also be used as the guide wire, in which case a dedicated guide wire need not be inserted previously but the catheter tube may be inserted once and for all to relieve the patient of unnecessary pain.

Claims:

(1)    A catheter tube characterized in that the catheter tube comprises a tube main body,

an inflatable first balloon placed around an outer peripheral wall of the tube in the vicinity of a distal end of the tube main body,

a first lumen formed in the tube main body and communicating with an interior of said first balloon,

a tubular member provided in the vicinity of a distal end thereof with an inflatable second balloon and having a passage communicating with exterior and interior of said second balloon, and

a second lumen formed in said tube main body and adapted for accommodating said tubular member,

wherein said tubular member moves axially within said second lumen with said second balloon remaining in the deflated state so that said second balloon can be moved to a position spaced a predetermined distance from the distal end of said tube main body.

(2)    The catheter tube according to claim 1, wherein the constituent material of said tubular member is an ultraelastic alloy.

(3)    The catheter tube according to claims 1 or 2, wherein said tubular member is used as the guide wire for said catheter tube.

(4)   A catheter tube characterized in that the catheter tube comprises tube main body,

an inflatable first balloon place around an outer peripheral wall of the tube in the vicinity of a distal end of the tube main body,

a lumen formed in said tube main body, said lumen communicating with the inside of said first balloon and opening into the foremost part of the tube main body, and

a tubular member accommodated within said lumen, said tubular member having an inflatable second balloon in the vicinity of the distal end thereof and a passage communicating with the interior of said second balloon,

wherein said tubular member moves axially within said lumen with said second balloon remaining in the deflated state so that said second balloon can be moved to a position spaced a predetermined distance from the distal end of said tube main body.

(5)   The catheter tube according to any one of claims 1 to 4, wherein said catheter tube is a catheter tube for blood vessel.

(6)   An endoscope characterized in that the tube main body of the catheter tube according to any one of claims 1 to 5 is provided with a lumen adapted for accommodating an observation instrument, and the observation instrument is accommodated within said lumen.

Claims:

(1)  A catheter tube characterized in that the catheter tube comprises a tube main body,

an inflatable first balloon placed around an outer peripheral wall of the tube in the vicinity of a distal end of the tube main body,

a first lumen formed in the tube main body and communicating with an interior of said first balloon,

a tubular member provided in the vicinity of a distal end thereof with an inflatable second balloon and having a passage communicating with exterior and interior of said second balloon, and

a second lumen formed in said tube main body and adapted for accommodating said tubular member,

wherein said tubular member moves axially within said second lumen with said second balloon remaining in the deflated state so that said second balloon can be moved to a position spaced a predetermined distance from the distal end of said tube main body, and a space for observation having an arbitrary length is definable between said first balloon and said second balloon.

(2)  The catheter tube according to claim 1, wherein the constituent material of said tubular member is an ultraelastic alloy.

(3)    The catheter tube according to claims 1 or 2, wherein said tubular member is used as the guide wire for said catheter tube.

FIG. 1

FIG. 2

# F I G. 3

F I G. 4

F I G. 5

F I G. 6

# INTERNATIONAL SEARCH REPORT

International Application No PCT/JP89/00134

## I. CLASSIFICATION OF SUBJECT MATTER (if several classification symbols apply, indicate all) 6

According to International Patent Classification (IPC) or to both National Classification and IPC

Int. Cl$^4$ A61B1/00

## II. FIELDS SEARCHED

Minimum Documentation Searched 7

| Classification System | Classification Symbols |
|---|---|
| IPC | A61B1/00, A61M25/00 |

Documentation Searched other than Minimum Documentation
to the Extent that such Documents are Included in the Fields Searched 8

| Jitsuyo Shinan Koho | 1969 - 1989 |
| Kokai Jitsuyo Shinan Koho | 1971 - 1989 |

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| X, Y | JP, A, 51-11689 (Fuji Photo Optical Co., Ltd.) 29 January 1976 (29. 01. 76) Page 1, lower right column, line 8 to page 2, upper left column, line 15 (Family: none) | 1 |
| Y | JP, A, 59-181121 (Olympus Optical Co., Ltd.) 15 October 1984 (15. 10. 84) Page 3, lower left column, line 8 to lower right column, line 6 (Family: none) | 3, 5 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| April 26, 1989 (26. 04. 89) | May 15, 1989 (15. 05. 89) |

| International Searching Authority | Signature of Authorized Officer |
|---|---|
| Japanese Patent Office | |

Form PCT ISA/210 (second sheet) (January 1985)